# EUROPÄISCHE PATENTSCHRIFT

(11) **EP 2 417 954 B1**
(45) Veröffentlichungstag und Bekanntmachung des Hinweises auf die Patenterteilung: **03.07.2013**
(21) Anmeldenummer: 11007729.4
(22) Anmeldetag: 09.09.2010
(51) Int. Cl.: A61F 13/20

(54) **Tampon**
Tampon
Tampon

(30) Priorität: 09.09.2009 DE 202009012237 U
(43) Veröffentlichungstag der Anmeldung: 15.02.2012
(62) Teilanmeldung aus: 10009402.8
(73) Patentinhaber: Rauscher Consumer Products GmbH, 1140 Wien (AU)
(72) Erfinder: Steinlechner, Erik, 2500 Baden (AT); Pötsch, Ernst, 2525 Schönau (AT); Mikulaschek, Marlene Sophie, 1230 Wien (AT); Posch, Karl-Heinz, 2801 Katzelsdorf (AT); Süßle, Wolfgang, 2500 Baden (AT)
(74) Vertreter: Leinweber & Zimmermann

(56) Entgegenhaltungen:
- GB-A- 2 056 283
- GB-A- 2 073 592
- US-A- 2 386 590
- US-A- 4 018 225
- US-A1- 2004 030 280

## Beschreibung

Die Erfindung betrifft ein Tampon zum Einführen in eine Körperhöhle, insbesondere in die weibliche Scheide, mit einem im wesentlichen zylindrischen Saugkörper, der eine sich in Richtung auf ein axiales Ende davon verjüngende Kuppe aufweist.

Tampons der gerade beschriebenen Art werden zur Aufnahme von Körperflüssigkeit in der Wundversorgung, maßgeblich aber im Zusammenhang mit der weiblichen Menstruation eingesetzt. Dabei trifft die Menstruationsflüssigkeit von außen auf den Tampon und dringt von außen nach innen in den Saugkörper ein. Demnach gelangt die Flüssigkeit durch die äußeren Tamponschichten in den üblicherweise verdichteten Tamponkern, welcher das größte Absorptionsvermögen aufweist. Allerdings besteht bei der Verwendung herkömmlicher Tampons das Risiko, daß die äußeren Schichten des Tampons bereits mit Flüssigkeit gesättigt sind, bevor die Flüssigkeit zum Tamponkern vordringt und dann die Gefahr des Auslaufens von Flüssigkeit auftritt, obwohl der Saugkern des Saugkörpers noch nicht mit Flüssigkeit gesättigt bzw. vollständig benutzt ist.

Zur Behebung dieses Mangels wurden bereits Tampons vorgeschlagen, deren Saugkörper sich parallel zur Zylinderachse erstreckende Rillen in ihrer äußeren Begrenzungsfläche aufweisen. Zur Verbesserung der Absorptionseigenschaften von Tampons wurde auch vorgeschlagen, diese Rillen wellenförmig und/oder wendelförmig die Zylinderachse des im wesentlichen zylindrischen Saugkörpers umlaufend auszuführen.

Wenngleich mit entsprechend ausgeführten Tampons eine Verbesserung der Absorptionseigenschaften erreicht werden kann, hat es sich gezeigt, daß weiterhin das Auslaufen von Körperflüssigkeit aus der mit dem Tampon zu verschließenden Körperhöhle beobachtet wird, obwohl das Absorptionsvermögen des Tampons noch nicht vollständig ausgenutzt ist.

In der US 4,318,405 ist ein Tampon mit einer sich in Richtung auf ein axiales Ende verjüngenden Kuppe beschrieben, bei dem eine Kapsel zum Einbringen pharmazeutisch wirksamer Substanzen in eine Vertiefung der Kuppe eingesetzt ist. Mit diesen Tampons können therapeutisch wirksame Substanzen eingebracht werden. Eine Verbesserung im Hinblick auf die Aufnahme von aus der Körperhöhle auslaufender Körperflüssigkeit wird durch Einsatz dieser Tampons allerdings nicht erreicht.

In der US 4,077,408 ist ein Tampon beschrieben, der aus einem in einer wasserlöslichen Hülle aufgenommenen Schaumkern besteht. Dadurch soll ein ausreichendes Ausdehnungsvermögen des Tampons während des Gebrauchs sichergestellt werden. Allerdings muß dazu erst die wasserlösliche Hülle aufgelöst werden, was wiederum zu Problemen hinsichtlich des Auslaufens von Körperflüssigkeit führen kann.

Ähnliche Tampons sind in der US 4,088,132 beschrieben. Auch diese Tampons sind im Hinblick auf die zuverlässige Vermeidung des Auslaufens von Körperflüssigkeit problematisch.

Endlich sind in der US 3,343,225 und US 3,515,138 Tampons nach dem Oberbegriff des Patentanspruchs 1 beschrieben, bei denen eine Vertiefung in der Kuppe des Tampons vorgesehen ist, um so die Oberfläche und das Saugvermögen des Tampons zu verbessern. Bei diesen bekannten Tampons ist der Übergang zwischen dem im wesentlichen zylinderförmigen Saugkörper und der Stirnfläche des Tampons abgerundet, wobei sich die trichterförmige Vertiefung in der Stirnfläche des Tampons im wesentlichen über die gesamte Stirnfläche bis zu dem abgerundeten Übergangsbereich erstreckt.

In der GB 2073592 A ist ein Tampon mit einem sich von einem ebenen oder konkav gewölbten Einführende in Richtung auf das entgegengesetzte und den Rückholfaden tragende Ende erstreckenden Hohlraum angegeben, ei dem in dem ebenen Einführende radial verlaufende und in den Hohlraum mündende Kanäle gebildet sein können.

In der US 2004/0030280 A1 ist ein Tamponsystem angegeben, bei dem eine in einem Einführende des Tampons vorgesehene Öffnung über einen Kanal bzw. einen Sammelraum mit einem in dem Tampon eingebetteten saugfähigen Material in Verbindung steht.

In der US 2,385,590 ist ein Tampon mit einer im Einführende vorgesehenen nadelartigen Ausnehmung beschrieben, wobei in dem Einführende eine Anzahl von Falten oder einschnitten vorgesehen sein kann, die in die nadelartige Ausnehmung münden.

Es hat sich allerdings gezeigt, daß die mit diesen Tampons angestrebte Verbesserung des Saugvermögens mit der beschriebenen Tampongeometrie nicht ohne Beeinträchtigung der Gebrauchseigenschaften erreicht werden kann und weiterhin das Auslaufen von Körperflüssigkeit aus der Körperhöhle beobachtet wird, selbst wenn das Absorptionsvermögen des Tampons noch nicht vollständig ausgenutzt ist.

Angesichts dieser Probleme im Stand der Technik liegt der Erfindung die Aufgabe zugrunde, einen Tampon bereitzustellen, dessen Absorptionsfähigkeit ohne Beeinträchtigung der sonstigen Gebrauchseigenschaften optimal ausgenutzt werden kann.

Erfindungsgemäß wird diese Aufgabe durch eine Weiterbildung bekannter Tampons gelöst, die im wesentlichen dadurch gekennzeichnet ist, daß der Durchmesser der Vertiefung an ihrem dem Scheitelbereich zugewandten Rand 20 bis 40 %, besonders bevorzugt etwa 25 bis 35 % des Saugkörperdurchmessers beträgt, und das Volumen der Vertiefung 4 % oder weniger des Gesamtvolumens des Saugkörpers entspricht.

Die Erfindung geht auf die Erkenntnis zurück, daß die mangelhaften Absorptionseigenschaften herkömmlicher Tampons darauf zurückzuführen sind, daß gerade im Kuppenbereich eine rasche Sättigung in den Randschichten des Tampons auftritt, die dann dazu führt, daß die Körperflüssigkeit am Tampon insgesamt vorbeiströmt und aus der zu verschließenden Körperhöhe ausläuft. Durch die erfindungsgemäße Weiterbildung wird im Kuppenbereich ein Trichter bzw. Saugkanal zur Verfügung gestellt, durch den die dort auftreffende Körperflüssigkeit, insbesondere Menstruationsflüssigkeit, direkt in den Saugkern des Saugkörpers gelangt, so daß eine frühzeitige Sättigung des äußeren Bereichs des Saugkörpers im Kuppenbereich zuverlässig verhindert werden kann und auch der innere Bereich des Tampons optimal genutzt werden kann. Dabei hat sich im besonderen gezeigt, daß durch die Bereitstellung der Vertiefung, d. h. eines konkaven Oberflächenbereichs im Bereich der ansonsten konvexen Tamponkuppe keine nennenswerten Beeinträchtigungen der Einführeigenschaften des Tampons, welche durch die Kuppe verbessert werden sollen, beobachtet werden. Insgesamt wird durch die erfindungsgemäße Weiterbildung von Tampons also die Absorptionseigenschaft des Tampons verbessert, ohne dessen sonstige Gebrauchseigenschaften zu beeinträchtigen.

Dabei ist der Durchmesser der Vertiefung an ihrem dem Scheitelbereich zugewandten Rand, d. h. der größte Durchmesser der Vertiefung in einer senkrecht zur Zylinderachse des Saugkörpers verlaufenden Richtung, so gewählt, daß er weniger als die Hälfte des Durchmessers des Saugkörpers beträgt. Diese Begrenzung des Vertiefungsdurchmessers geht auf die Erkenntnis zurück, daß die bei Tampons gemäß der US 3,343,225 beobachteten Probleme auf ein Zusammendrücken des Tampons im Bereich der Vertiefung zurückzuführen sind, wodurch die angestrebte Oberflächenvergrößerung und die gewünschte Verbesserung des Saugvermögens während des Gebrauchs verhindert wird. Wenn der Durchmesser der Vertiefung gemäß der vorliegenden Erfindung auf einen Wert von weniger als 50 % des Durchmessers des Saugkörpers eingestellt wird, kann eine ausreichende Stabilität des Tampons im Bereich der mit der Vertiefung versehenen Kuppe sichergestellt werden, so daß die angestrebte Oberflächenvergrößerung und die damit erreichte Verbesserung der Einleitung von Körperflüssigkeit in den Tamponkern zuverlässig erreicht wird, auch wenn der Tampon während des Gebrauchs einer Radialkraftbeaufschlagung unterzogen wird.

Falls der Durchmesser weniger als 20 % des Saugkörperdurchmessers beträgt, werden Beeinträchtigungen der gewünschten Eigenschaften hinsichtlich der Einleitung von Körperflüssigkeit in den Saugkern beobachtet. Bei einer Vergrößerung des Durchmessers der Vertiefung auf einen Wert von mehr als 40 % des Saugkörperdurchmessers kommt es zu Beeinträchtigungen bei der Einführung des Tampons in die Körperhöhle. Die erfindungsgemäße trichterförmige Verjüngung der Vertiefung hat sich sowohl aus fertigungstechnischen Gründen als auch im Hinblick auf die gewünschte optimale Ausnutzung der Absorptionseigenschaften des Tampons als zweckmäßig erwiesen.

Durch die Bereitstellung der Vertiefung in dem Tampon kann sich eine Veminderung der Menge absorptionsfähigen Materials des Tampons insgesamt ergeben. Im Hinblick auf die Optimierung der Ausnutzung der Absorptionsfähigkeit entspricht das Volumen der Vertiefung 4 % oder weniger, vorzugsweise 2 % oder weniger, besonders bevorzugt 1 % oder weniger, insbesondere 0,75 % oder weniger des Gesamtvolumens des Tampons. Dabei wird der gewünschte Effekt der Verbesserung der Einleitung von Körperflüssigkeit in den Tamponkern noch sichergestellt, wenn das Volumen der Vertiefung 0,1 % oder mehr, insbes. 0,2 % oder mehr, vorzugsweise 0,5 % oder mehr des Gesamtvolumens des Tampons entspricht.

Aus fertigungstechnischer Sicht hat es sich als günstig erwiesen, wenn die erfindungsgemäß in der Tamponkuppe vorgesehene Vertiefung eine mindestens 2-zählige Drehsymmetrie bezüglich einer etwa kolinear zur Längsachse des Saugkörpers verlaufenden Symmetrieachse aufweist, also beispielsweise rechteckförmig ist. Im Hinblick auf eine gute Ausnutzung der Absorptionseigenschaften des Tampons ist es besonders zweckmäßig, wenn die Vertiefung eine 3-zählige Symmetrie mit dreieckförmigem Querschnitt, eine 4-zählige Symmetrie mit quadratischem Querschnitt und besonders bevorzugt eine Rotationssymmetrie mit kreisförmigem Querschnitt aufweist.

Im Hinblick auf die Optimierung der Einleitung von Körperflüssigkeit in den Saugkern des Saugkörpers hat es sich als zweckmäßig erwiesen, wenn sich die Vertiefung über mindestens 20 % insbesondere mindestens 50 % der axialen Länge der Kuppe erstreckt, die Kuppe vorzugsweise in axialer Richtung vollständig durchsetzt und besonders bevorzugt ausgehend von dem umlaufenden Rand über die Kuppe hinaus in den Saugkern hineinreicht. Dabei kann der Durchmesser der Vertiefung an ihrem die Zylinderachse umlaufenden Rand etwa 10 bis 45 %, insbesondere 20 bis 40 %, vorzugsweise etwa 25 bis 35 % des Saugkörperdurchmessers betragen.

Ähnlich wie bei herkömmlichen Tampons kann auch ein erfindungsgemäßer Tampon ein eine Mantelfläche des Saugkörpers abdeckendes Hüllmaterial, wie etwa ein auf Watte aufsiegelbares Hüllvlies oder eine vorzugsweise perforierte Hüllfolie, aufweisen, mit dem verhindert wird, daß Tamponfasern sich aus dem Tamponkörper lösen, wenn der Tampon sich im Rahmen der Aufnahme von Körperflüssigkeit ausdehnt. Im Rahmen der Erfindung einsetzbare Hüllmaterialien sind bspw. in der DE 69811114 T2, der EP 1194100 B1, der EP 925050 B1 und der US 68860874 beschrieben. Der Offenbarungsgehalt dieser Schriften wird hiermit durch ausdrückliche Inbezugnahme hinsichtlich der Beschaffenheit des Hüllmaterials in diese Beschreibung aufgenommen. Ferner können auch bei erfindungsgemäßen Tampons zwei, drei oder mehr geradlinig, wellenförmig und/oder wendelförmig verlaufende Rillen in der Mantelfläche des Saugkörpers vorgesehen sein, um die Absorptionseigenschaften zu verbessern. Üblicherweise sind Tampons für den Transport und die Lagerung in einer Kunststofffolie aufgenommen. Dabei kommen regelmäßig Zellophanfolien zum Einsatz. Im Rahmen der Erfindung hat es sich gezeigt, daß das Ablösen von einzelnen Fasern im Bereich des Randes der Vertiefung bei dem Ablösen der Verpakkungsfolie von dem Tampon gut verhindert werden kann, wenn anstelle üblicher Zellophanfolien eine OPP-Folie, also eine Polypropylenfolie zum Verpacken der Tampons eingesetzt wird.

Herkömmliche Tampons werden hergestellt, indem zunächst ein Hüllvlies auf ein Watteband aufgesiegelt wird, so daß das Hüllvlies sich über die Hälfte der Länge eines Wattebandstreifens erstreckt, der für die Herstellung eines Tampons benötigt wird und über den Wattestreifen hinausreicht. Beim nächsten Herstellungsschritt wird aus dem Wattestreifen ein Wattewickel gebildet, bei dem das Hüllvlies eine äußere Begrenzungsfläche des Wickels bildet, welcher den Wattestreifen überlappt und auf eine vorherige Wickellage des Hüllvlieses aufgesiegelt werden kann, um so eine umlaufende äußere Begrenzungsfläche des Wattewickels zu bilden. Anschließend wird der Wickel radial und/oder linear verpreßt, insbesondere um so einen etwa zylindrischen Saugkörper bereitzustellen. Im Rahmen der radialen Verpressung werden auch die ggf. vorzusehenden Rillen in der Mantelfläche des Saugkörpers gebildet. Zur Fertigstellung des Tampons wird der bereits gepreßte Tamponrohling in Formwerkzeuge einer Kuppenformeinrichtung eingeführt, deren Form der gewünschten Kuppenform entspricht.

Eine Vorrichtung zur Herstellung eines erfindungsgemäßen Tampons mit einer Kuppenformeinrichtung zum Formen einer Kuppe an einem im wesentlichen zylindrischen und bereits gepreßten Tamponrohling, bei dem die Kuppenformeinrichtung mindestens ein Formwerkzeug mit einem beim Formvorgang in Richtung der Zylinderachse des Rohlings in den Rohling einführbaren Dorn aufweist, um so die das Einleiten der Menstruationsflüssigkeit in den Saugkörperkern begünstigende Vertiefung bzw. den entsprechenden trichterförmigen Einleitungskanal in der Tamponkuppe zu bilden, ist im wesentlichen dadurch gekennzeichnet, daß das Formwerkzeug einen ringförmigen Mantel mit sich zumindest abschnittweise in axialer Richtung verjüngendem Innendurchmesser und einem in den Mantel einsetzbaren Dorn aufweist.

Das Formwerkzeug ist demnach modular aufgebaut. Dabei wird durch den Mantel die äußere konvexe und das Einführen des Tampons in die Körperhöhle begünstigende Oberfläche der Kuppe gebildet, während durch den in den Mantel eingesetzten Dorn die Vertiefung gebildet wird, mit der die Einleitung von Menstruationsflüssigkeit in den Saugkörperkern begünstigt wird. Durch den modularen Aufbau kann erreicht werden, daß bei einem mehrstufigen Herstellungsprozeß, bei dem der Saugkörper nacheinander in eine Anzahl von Werkzeugen eingesetzt wird unter Verwendung ein und desselben Mantels unterschiedliche Dornformen eingesetzt werden können. Bei dem beschriebenen modular aufgebauten Formwerkzeug kann der Erhalt der gewünschten Kuppenform besonders zuverlässig sichergestellt werden, wenn zwischen dem den sich verjüngenden Innendurchmesser aufweisenden Bereich der inneren Begrenzungsfläche des Mantels und dem Dorn ein Ringspalt freigelassen ist und der Mantelinnenraum an einem dem Dorn abgewandten axialen Ende mit einem vorzugsweise einstückig mit dem Dorn ausgeführten Dornträger verschlossen ist. Gegebenenfalls kann der Dorn in axialer Richtung über den dem Dornträger abgewandten Rand des Mantels hinausragen, um so eine die Kuppe vollständig durchsetzende und ggf. über die Kuppe hinausragende Vertiefung in der Kuppe zu bilden. Wie vorstehend bereits angedeutet, kann die Kuppenformeinrichtung zwei, drei oder mehr Formwerkzeuge aufweisen, in die die Rohlinge beim Formvorgang nacheinander einführbar sind.

Im Hinblick auf die Vereinfachung des Herstellungsvorgangs beim Einsatz mehrerer Formwerkzeuge sind diese zweckmäßigerweise an einem gemeinsamen Träger angeordnet, der bezüglich einer Drehachse drehbar gelagert ist, wobei die Formwerkzeug auf einer die Drehachse umlaufenden Kreislinie angeordnet sind. Dann können die Formwerkzeug durch Drehen des Trägers in dem Rohling gegenüberliegende Position gebracht werden und durch eine axiale Relativbewegung zwischen Rohling und Formwerkzeug der gewünschte Formgebungsvorgang eingeleitet werden.

Ein Formwerkzeug für eine Vorrichtung zum Herstellen erfindungsgemäßer Tampons ist im wesentlichen dadurch gekennzeichnet, daß es einen ringförmigen Mantel mit einem darin eingesetzten Dorn aufweist, wobei der Dorn zweckmäßigerweise austauschbar in dem Mantel aufgenommen ist. Ein erfindungsgemäßer Tampon wird, wie der vorstehenden Erläuterung zu entnehmen ist, hergestellt, in dem ein im wesentlichen zylindrischer und bereits radial und/oder linear gepreßter Tamponrohling in das Formwerkzeug in axialer Richtung eingeführt wird.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert.

In der Zeichnung zeigt:
- **Fig. 1**: eine schematische Darstellung eines erfindungsgemäßen Tampons,
- **Fig. 2**: eine schematische Darstellung einer Vorrichtung zum Herstellen erfindungsgemäßer Tampons und
- **Fig. 3**: schematische Darstellungen von Formwerkzeugen zum Herstellen erfindungsgemäßer Tampons.

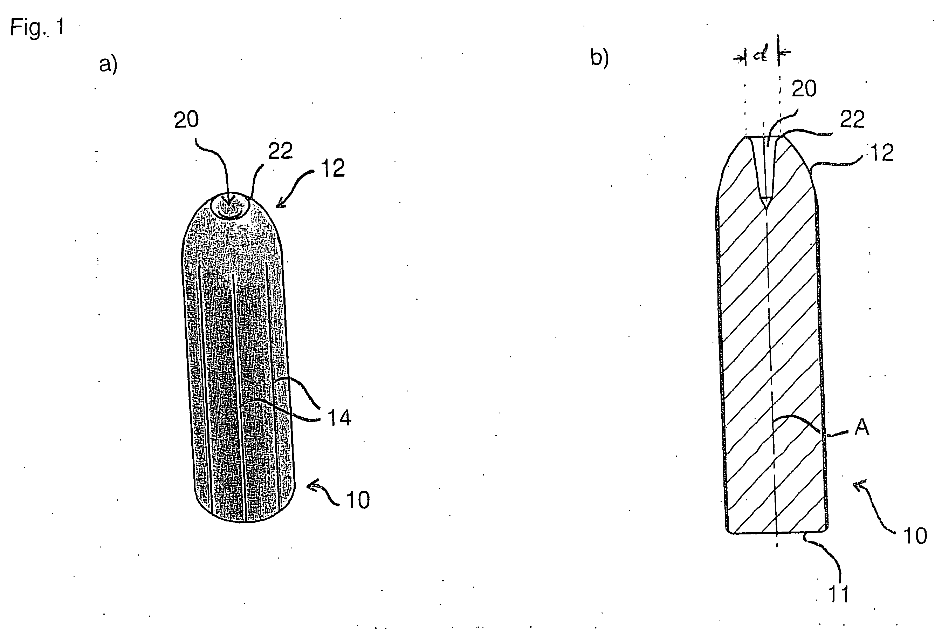

Gemäß Fig. 1 umfaßt der erfindungsgemäße Tampon einen insgesamt mit 10 bezeichneten im wesentlichen zylindrischen Saugkörper, der an einem seiner axialen Enden eine sich in Richtung auf ein axiales Ende davon verjüngende Kuppe 12 aufweist. Der Saugkörper 10 weist in seiner Mantelfläche eine Anzahl von parallel zueinander und parallel zur Zylinderachse A des Saugkörpers verlaufenden Rillen 14 auf. Im Scheitelbereich der Kuppe 12 ist eine sich ausgehend von einem die Zylinderachse A umlaufenden Rand 22 in Richtung auf ein der Kuppe 12 abgewandtes axiales Ende 11 des Saugkörpers 10 erstrekkende trichterförmige Vertiefung 20 gebildet, welche bei dem in der Zeichnung dargestellten Ausführungsbeispiel der Erfindung rotationssymmetrisch bezüglich der Zylinderachse A ausgeführt ist. Wie besonders deutlich der Fig. 1 b zu entnehmen ist, verjüngt sich der Querschnitt der Vertiefung 20 ausgehend vom Rand 22 in Richtung auf das der Kuppe 11 abgewandte axiale Ende des Saugkörpers 10.

Der Durchmesser "d" der Vertiefung an ihrem dem Scheitelbereich zugewandten Rand (22), d. h. der größte Durchmesser der Vertiefung in einer sich senkrecht zur Zylinderachse "A" erstreckenden Richtung, beträgt bei der in der Zeichnung dargestellten Ausführungsform der Erfindung etwa 30 % des Saugkörperdurchmessers.

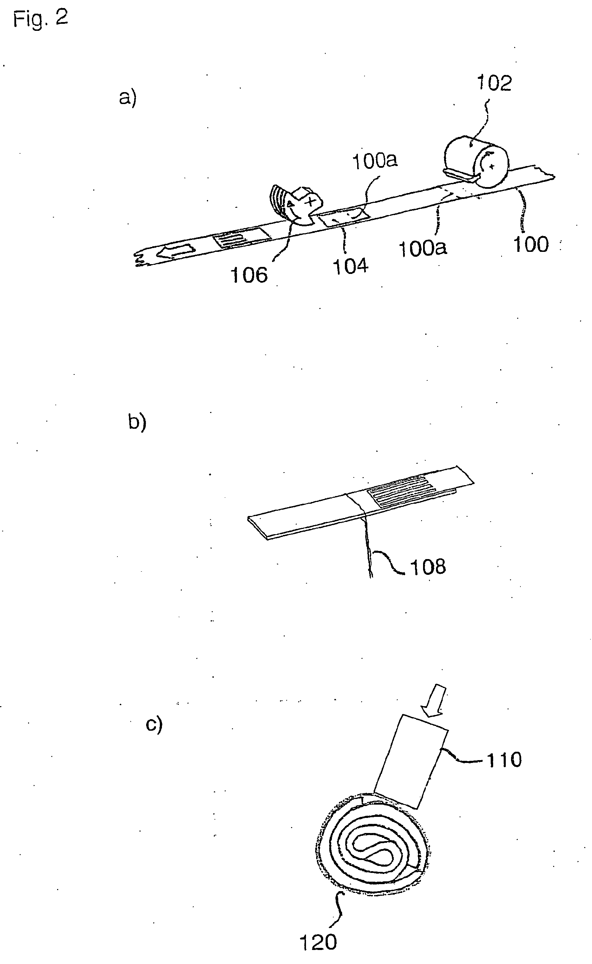

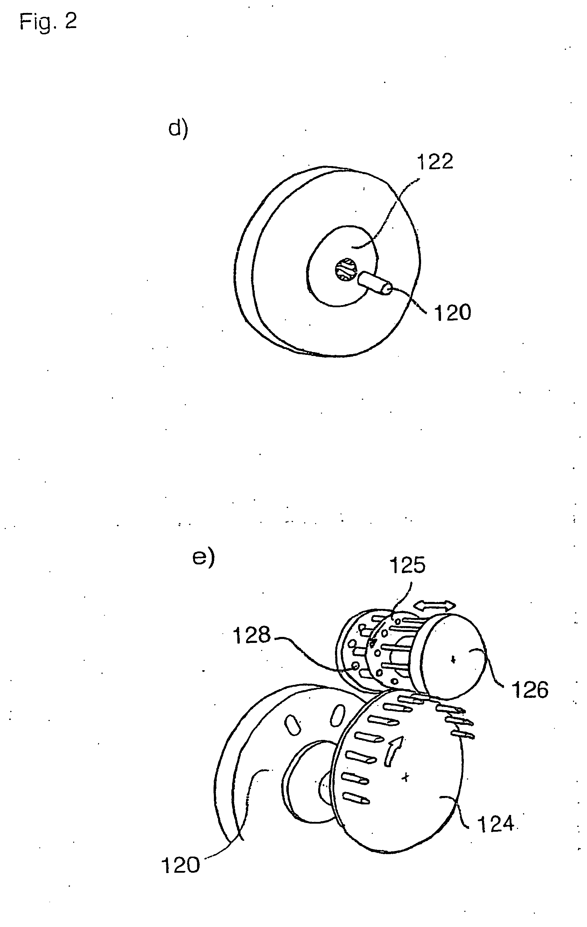

Tampons gemäß Fig. 1 können mit den nachstehend anhand der Fig. 2 erläuterten Vorrichtungen hergestellt werden. Gemäß Fig. 2 wird bei der Herstellung eines erfindungsgemäßen Tampons zunächst ein Watteband 100 in eine Tamponmaschine eingezogen und mit einem Wattemesser 102 auf die zur Herstellung einzelner Tampons benötigte Länge geschnitten, wie in Fig. 2a anhand der strichlinierten Linien 100a angedeutet.

Nach Schneiden des Wattebands zu Wattestreifen mit der benötigten Länge wird ein Hüllvlies 104 mit etwa der halben Länge der Wattestreifen versetzt um die halbe Länge eines Wattebandstreifens unter Druck und Hitze mit Hilfe einer Siegelbacke 106 auf das Watteband 100 aufgesiegelt. Das Hüllvlies ragt in Längsrichtung über den Wattestreifen hinaus, auf dem es aufgesiegelt ist. In einem nächsten Herstellungsschritt wird ein Rückholfaden 108 um den Wattestreifen 100 geschlungen und verknotet, wie in Fig. 2b dargestellt. Der Wattestreifen 100 wird zu einem sogenannten Wickel gerollt, derart daß der über den Wattestreifen hinausragende Bereich des Hüllvlies auf dem Wickel aufliegt und mit der vorhergehenden Hüllvlies-Wickellage versiegelt werden kann. Dazu wird eine Siegelbacke 110 eingesetzt, wie in Fig. 2c schematisch angedeutet.

Der so vorbereitete Wattewickel 120 wird an eine geöffnete Presse 122 übergeben, wie in Fig. 2d angedeutet. Durch Schließen der Presse 122 wird ein Rohling mit Rillen und entsprechendem Durchmesser (abhängig von der Größe, z. B. Mini, Normal, Super...) und anderen einstellbaren Parametern gepreßt.

Nach leichtem Anlüften der Presse 120 wird der damit erhaltene gepreßte und im wesentlichen zylinderförmige Rohling mit einem Ausschieber nach hinten aus der Presse gedrückt und an eine Röhrchentrommel 124 übergeben. In einer Kopftrommel 125 wird der Rohling mit Stößeln 126 in vorgegebener Reihenfolge in Formwerkzeuge einer Kuppenformeinrichtung gedrückt. Abhängig von der gewünschten Kuppenform und Ausführung des Tampons sind in der Kuppenformeinrichtung 128 4 bis 12 gleiche oder auch unterschiedliche Formwerkzeuge eingebaut.

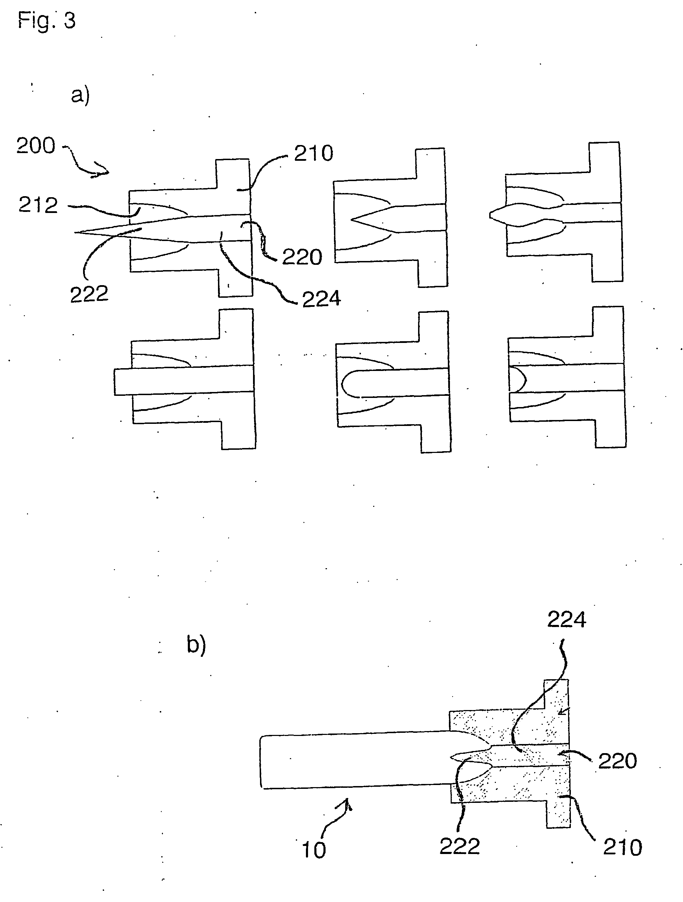

Die in einer Vorrichtung zur Herstellung erfindungsgemäßer Tampons zum Einsatz kommenden Formwerkzeuge werden nachstehend anhand der Fig. 3 erläutert. Danach weisen die insgesamt mit 200 bezeichneten Formwerkzeuge einen ringförmigen Mantel 210 und eine Dornanordnung 220 auf. Die Dornanordnung besteht aus einem Dorn 222 und einem Dornträger 224. Der Mantel 210 weist eine innere Begrenzungsfläche 212 mit sich zumindest abschnittweise in axialer Richtung verjüngendem Innendurchmesser auf. Insgesamt ist der Mantel 210 rotationssymmetrisch ausgeführt. Der Dorn 222 ist derart in den Mantel 210 eingeführt, daß zwischen dem den sich verjüngenden Innendurchmesser enthaltenden Bereich der inneren Begrenzungsfläche 212 des Mantels und dem Dorn ein Ringspalt freigelassen ist und der Mantelinnenraum an seinem dem Dorn 222 abgewandten axialen Ende mit dem einstückig mit dem Dorn 222 gebildeten Dornträger 224 verschlossen ist. Gemäß Fig. 3a können bei Einsatz identisch ausgeführter Mantelelemente 210 unterschiedlich ausgeführte Dorne zum Einsatz kommen. Dabei können längere, kürze, dickere oder dünnere Dorne bzw. Dorne mit zylindrischem, bombiertem oder gewelltem Verlauf benutzt werden, wobei die Dornform entsprechend der gewünschten Form der Vertiefung 20 in der Kuppe des Tampons gewählt wird. Gemäß Fig. 3b wird der Tamponrohling in axialer Richtung in das Formwerkzeug 200 eingeführt, so daß der Dorn 222 in axialer Richtung in den Tampon eindringt, während gleichzeitig der äußere Kuppenring gebildet wird. Bei der in der Zeichnung dargestellten Ausführungsform der Erfindung beträgt das Volumen der Vertiefung ausgehend von dem umlaufenden Rand 22 bis zum der Kuppe 12 abgewandten axialen Ende 11 des Saugkörpers zugewandten Boden etwa 0,54 % des Gesamtvolumens des Tampons. Dabei kann sich der Durchmesser der Vertiefung ausgehend von einem Randdurchmesser im Bereich von etwa 5 mm ggf. über mehrere Stufen verjüngen und die Vertiefung in einer Spitze auslaufen, wie besonders deutlich in Fig. 1 b zu erkennen ist, so daß ein in einen Kanal mündender Trichter gebildet wird, durch den Menstruationsflüssigkeit in den Kern des Saugkörpers 10 eingeleitet werden kann.

Die Erfindung ist nicht auf das anhand der Zeichnung erläuterte Ausführungsbeispiel beschränkt. Vielmehr ist auch daran gedacht, Tampons mit unterschiedlicher Form der Rillen 14 und/oder unterschiedlicher Querschnittsform der Vertiefung 20 einzusetzen. Es können Vertiefungen mit beliebigem, insbesondere dreieckigem, quadratischem oder rechtekkigem Querschnitt zum Einsatz kommen. Bei dem anhand der Zeichnung erläuterten Ausführungsbeispiel der Erfindung durchsetzt die Vertiefung 20 die Kuppe 12 vollständig. Bei anderen Ausführungsformen der Erfindung kann die Vertiefung 20 über die Kuppe hinaus in den Saugkörperkern hineinreichen. In einigen Fällen ist es auch günstig, wenn die Vertiefung 20 nur einen Teil der Kuppe 12 durchsetzt. Zur Herstellung erfindungsgemäßer Tampons werden Tamponrohlinge in ein Formwerkzeug eingeführt. Dabei kann die Vertiefung in mehreren Schritten ausgeführt werden, indem der Tamponrohling nacheinander in identisch oder unterschiedlich ausgeführte Formwerkzeuge ausgeführt wird. Die Formwerkzeuge können dabei auf einer Formwerkzeugtrommel angeordnet sein, welche durch Drehen um eine Drehachse in eine dem Tampon gegenüberliegende Stellung gebracht wird, so daß der Tampon durch axiale Relativbewegung zwischen Formwerkzeug und Rohling nacheinander in unterschiedliche Formwerkzeuge eingeführt werden kann.

## Patentansprüche

1. Tampon zum Einführen in eine Körperhöhle, insbesondere in die weibliche Scheide, mit einem im wesentlichen zylinderförmigen Saugkörper (10), der eine sich in Richtung auf ein axiales Ende verjüngende Kuppe (12) aufweist, bei dem in einem ein axiales Ende der Kuppe (12) bildenden Scheitelbereich davon eine sich ausgehend von einem die Zylinderachse (A) umlaufenden Rand davon in Richtung auf das der Kuppe (12) abgewandte Ende (11) des Saugkörpers (10) erstreckende und sich in Richtung auf das der Kuppe (12) abgewandte axiale Ende (11) des Saugkörpers (10) trichterförmig verjüngende Vertiefung (20) gebildet ist, **dadurch gekennzeichnet, daß** der Durchmesser der Vertiefung (20) an ihrem dem Scheitelbereich zugewandten Rand (22) etwa 20 bis 40 %, besonders bevorzugt etwa 25 bis 35 % des Saugkörperdurchmessers beträgt, und das Volumen der Vertiefung 4 % oder weniger des Gesamtvolumens des Saugkörpers (10) entspricht.

2. Tampon nach Anspruch 1, **dadurch gekennzeichnet, daß** die Vertiefung (20) eine mindestens 2-zählige Drehsymmetrie bezüglich einer etwa kolinear zur Längsachse des Saugkörpers (10) verlaufenden Symmetrieachse (A) aufweist.

3. Tampon nach Anspruch 1 oder 2, **dadurch gekennzeichnet, daß** die Vertiefung sich über mindestens 20 %, vorzugsweise mindestens 50 % der axialen Länge der Kuppe (12) erstreckt, die Kuppe vorzugsweise in axialer Richtung vollständig durchsetzt.

4. Tampon nach einem der vorhergehenden Ansprüche, **dadurch gekennzeichnet, daß** das Volumen der Vertiefung (20) 2 % oder weniger, besonders bevorzugt 1 % oder weniger, insbesondere 0,75 % oder weniger des Gesamtvolumens des Saugkörpers (10) entspricht.

5. Tampon nach einem der vorhergehenden Ansprüche, **dadurch gekennzeichnet, daß** das Volumen der Vertiefung (20) 0,1 % oder mehr, insbesondere 0,25 % oder mehr, vorzugsweise 0,5 % oder mehr des Gesamtvolumens des Saugkörpers (10) beträgt.

6. Tampon nach einem der vorhergehenden Ansprüche, **dadurch gekennzeichnet, daß** zumindest eine Mantelfläche des Saugkörpers (10) ein auf Watte aufsiegelbares Hüllmaterial, wie etwa ein Hüllvlies (104) oder eine vorzugsweise perforierte Hüllfolie, aufweist.

7. Tampon nach einem der vorhergehenden Ansprüche, **dadurch gekennzeichnet, daß** die Mantelfläche des Saugkörpers zwei, drei oder mehr geradlinig, wellenförmig oder wendelförmig verlaufende Rillen (14) aufweist.

8. Tampon nach einem der vorhergehenden Ansprüche, **dadurch gekennzeichnet, daß** der Saugkörper (10) in einer Folie aus Polypropylen oder Zellophan aufgenommen ist.

## Claims

1. A tampon for insertion into a body cavity, in particular into the vagina, comprising a substantially cylindrical suction body (10) which has a point (12) tapering towards an axial end, wherein in an apex region of said point (12) forming an axial end of the latter a recess (20) extending from an edge of the cylinder axis (A) and passing round the latter towards the end (11) of the suction body (10) facing away from the point (12) and tapering in a funnel shape towards the axial end (11) of the suction body (10) facing away from the point (12) is formed, **characterised in that** the diameter of the recess (20) on its edge (22) facing towards the apex region is approximately 20 to 40 %, particularly preferably approximately 25 to 35 % of the suction body diameter, and the volume of the recess corresponds to 4 % or less of the overall volume of the suction body (10).

2. The tampon according to Claim 1, **characterised in that** the recess (20) has at least a 2-figure rotational symmetry in relation to an axis of symmetry (A) running approximately collinearly to the longitudinal axis of the suction body (10).

3. The tampon according to Claim 1 or 2, **characterised in that** the recess extends over at least 20 %, preferably at least 50 % of the axial length of the point (12), and preferably passes through the whole of the point in the axial direction.

4. The tampon according to any of the preceding claims, **characterised in that** the volume of the recess (20) corresponds to 2 % or less, particularly preferably 1 % or less, in particular 0.75 % or less of the overall volume of the suction body (10).

5. The tampon according to any of the preceding claims, **characterised in that** the volume of the recess (20) is 0.1 % or more, in particular 0.25 % or more, preferably 0.5 % or more of the overall volume of the suction body (10).

6. The tampon according to any of the preceding claims, **characterised in that** at least one lateral surface of the suction body (10) has a cladding material, such as for example a cladding fleece (104) or a preferably perforated cladding film that can be sealed onto cotton.

7. The tampon according to any of the preceding claims, **characterised in that** the lateral surface of the suction body has two, three or more grooves (14) running in a straight line, or in the form of waves or spirals.

8. The tampon according to any of the preceding claims, **characterised in that** the suction body (10) is held in a film made of polypropylene or cellophane.

## Revendications

1. Tampon destiné à être introduit dans un orifice corporel, notamment le vagin, comportant un corps absorbant (10) sensiblement cylindrique, qui présente une calotte (12) allant en rétrécissant dans la direction d'une extrémité axiale, dans lequel il est conçu, dans une partie de sommet de celui-ci constituant une extrémité axiale de la calotte (12), un renfoncement (20) qui s'étend depuis un bord du corps absorbant (10) en entourant l'axe (A) du cylindre en direction de l'extrémité axiale (11) du corps absorbant qui est opposée à la calotte, lequel renfoncement va en rétrécissant à la manière d'un entonnoir, **caractérisé en ce que** le diamètre du renfoncement (20) au niveau de son bord (22) tourné vers la partie de sommet fait d'environ 20 à 40 %, notamment de préférence d'environ 25 à 35 % du diamètre du corps absorbant, et **en ce que** le volume du renfoncement correspond à 4 % ou moins du volume total du corps absorbant (10).

2. Tampon selon la revendication 1, **caractérisé en ce que** le renfoncement (20) présente une symétrie en rotation qui est au moins double par rapport à un axe de symétrie (A) qui se prolonge de manière approximativement colinéaire à l'axe longitudinal du corps absorbant (10).

3. Tampon selon la revendication 1 ou 2, **caractérisé en ce que** le renfoncement se prolonge sur au moins 20 %, de préférence sur au moins 50 % de la longueur axiale de la calotte (12) en traversant complètement la calotte, de préférence dans la direction axiale.

4. Tampon selon l'une des revendications précédentes, **caractérisé en ce que** le volume du renfoncement (20) correspond à 2 % ou moins, notamment de préférence à 1 % ou moins, notamment à 0,75 % ou moins du volume total du corps absorbant (10).

5. Tampon selon l'une des revendications précédentes, **caractérisé en ce que** le volume du renfoncement (20) représente 0,1 % ou plus, notamment 0,25 % ou plus, de préférence 0,5 % ou plus du volume total du corps absorbant (10).

6. Tampon selon l'une des revendications précédentes, **caractérisé en ce qu'**au moins une surface d'enveloppe du corps absorbant (10) présente une matière d'enveloppement pouvant être scellée sur de la ouate, comme par exemple une étoffe d'enveloppement (104) ou une feuille d'enveloppement de préférence perforée.

7. Tampon selon l'une des revendications précédentes, **caractérisé en ce que** la surface d'enveloppe du corps absorbant présente deux, trois, ou plus de trois, rainures (14) droites, de forme ondulée ou hélicoïdale.

8. Tampon selon l'une des revendications précédentes, **caractérisé en ce que** le corps absorbant (10) est enserré dans une feuille de polypropylène ou de cellophane.
